# EUROPEAN PATENT SPECIFICATION

(11) **EP 0 508 488 B1**
(45) Date of publication and mention of the grant of the patent: **27.08.1997**
(21) Application number: 92107778.0
(22) Date of filing: 15.04.1987
(51) Int. Cl.: A61K 31/66

(54) **Use of inositol triphosphate for the treatment of inflammations**
Verwendung von Inositoltriphosphat zur Behandlung von Entzündungen
Utilisation de l'inositol-triphosphate pour le traitement d'inflammations

(30) Priority: 16.04.1986 SE 8601709
(43) Date of publication of application: 14.10.1992
(62) Divisional of application: 87105574.5
(73) Proprietor: PERSTORP AB, 28480 Perstorp (SE)
(72) Inventor: Sirén, Matti, F-00170 Helsinki (FI)
(74) Representative: Hansen, Bernd, Dr. Dipl.-Chem.

(56) References cited:
- EP-A- 0 179 439
- EP-A- 0 179 440
- EP-A- 0 179 441
- "Allgemeine und spezielle Pharmakologie und Toxikologie", 2nd edition, pages 590-594: "Schwermetalle", Bibliographisches Institut, Mannheim, DE
- NEUROCHEM. INT., vol. 10, no. 3, 1987, pages 361-369, Pergamon Journals Ltd, London, GB; G.Y. SUN et al.: "Labeling of phosphoinositides in rat brain membranes: An assessment of changes due to post-decapitative ischemic treatment"
- MED. SCI. RES., vol. 17, 16th - 30th September 1989, pages 749-750; C.J. GREEN et al.: "D-myo-inosital-1,2,6-trisphosphate (PP56) inhibits lipid peroxidation in warm ischaemic rabbit kidneys"

## Description

### FIELD OF INVENTION

The present invention relates to the use of inositol triphosphate in the preparation of a medicament for preventing or alleviating inflammatory conditions.

### BACKGROUND OF THE INVENTION

Even as early as the year 1900, different researchers had reported the finding of the organic phosphate compound phytic acid, i.e. 1.2.3.4.5.6-hexakis (dihydrogenphosphate) myo-inositol (also sometimes called inositol-hexaphosphoric acid) in plants. The content of phytic acid in different plants varies considerably. The content in grain is usually approximately 0.5-2%, with certain exceptions. Polished rice has a level of only 0.1% while wild rice contains as much as 2.2% phytic acid. Beans contain about 0.4-2%, oil plants approximately 2-5% and pollen 0.3-2%. The content of phytic acid in the plant varies during the growth period. The content is also influenced by, among other things, the climate.

In the literature there are reports on the presence of inositol pentaphosphate (IP₅) and inositol tetraphosphate (IP₄) in a few plants. It is further known that phosphate derivatives lower than IP₆ are formed at germination of grain. For instance the final products at the germination are inositol and phosphate. The use of IP₆ has been described in several scientific publications. The majority of the authors of these articles have observed several negative effects on humans and animals when consuming IP₆ or substances containing IP₆. Feeding dogs with too high an amount of IP₆ gives rise for example to rachitis. In humans lack of zinc and as a consequence thereof slower growth of children has been observed. Anemia has been observed mainly in women. Because of the above mentioned negative effects on the mineral balance in humans and animals, attempts have so far been made to reduce the intake of IP₆ and its derivatives to a minimum.

From C.A. Vol. 33 (1939), Abstr. No. 7351, No. 3/4 the use of phosphates including inositol phosphates as an anti-rachitic diet has been reported. No reference is made to specific inositol phosphates and nothing has been said in regard to inflammatory conditions.

U.S. patent 4,473,563 discloses the extracorporeal treatment of erythrocytes to incorporate therein inositol phosphates to improve the oxygen supply. Then erythrocytes are separated from drawn blood which has been pumped out of the body for that purpose. After complicated treatment of erythrocytes the latter are re-introduced into the blood. There is no disclosure of administering inositol phosphates directly to the body.

In U.S. patent 2,723,938 the use of inositol phosphates is disclosed for stabilizing dispersions of acqueous suspension of penicillin. This ensures that brief simple manual shaking will restore a state of complete and uniform dispersion of the penicillin after prolonged storage.

### SUMMARY OF THE INVENTION

According to the present invention it has quite unexpectedly been found that inositol triphosphate (IP₃), preferably in salt form, is useful to prevent or alleviate inflammatory conditions. Thus, the use of inositol triphosphate (IP₃), preferably in salt form, for preparing a medicament for preventing or alleviating inflammatory conditions has been provided by the present invention.

### DETAILED DESCRIPTION OF PREFERRED EMBODIMENTS

For production of the isomer or isomers of IP₃ which accomplish the above objective and which is present in the use according to the invention, one or more of the compounds IP₆, IP₅ or IP₄ or a natural product containing at least one of these compounds can be used as a starting material. In the cases where the starting material is a natural product, one with a content of at least 0.3%, preferably at least 1% of inositol phosphate (IP₆ + IP₅ + IP₄) is preferably chosen. Particularly suitable products are beans, bran, pollen and oil plants.

The composition used in the present invention should preferably contain at least 10%, most preferably at least 20%, or better yet at least 40% IP₃ calculated on the inositol content of the starting material. As high a level as possible of IP₃ in the composition is aimed at, as IP₃ has the best therapeutic effect according to experiments shown below. The IP₃ isomers present in the composition according to the invention can, for example, be produced by:
1) Enzymatic breakdown starting from IP₄, IP₅ and/or IP₆.
2) Chemical hydrolysis starting from IP₄, IP₅ and/or IP₆.
3) Chemical synthesis starting, for example, with inositol, IP₁, IP₂ and phosphate.
4) Enzymatic synthesis starting for example with from inositol, IP₁, IP₂ and phosphate.
5) Microbiological production (including also hybrid DNA-techniques).
6) Chemical or enzymatic migration of inositol phosphate or
7) Chemical or enzymatic hydrolysis of substituted inositol phosphate.

A combination of two or more of the above mentioned procedures may also be used.

According to the invention a procedure where the above mentioned higher inositol phosphates IP₆, IP₅ and/or IP₄ are broken down enzymatically to IP₃ with phytase enzyme, for instance, is preferred. Phytase enzyme is normally present in all inositol phosphate containing plants and seeds. Because of this it is, according to the invention, usually not necessary to add the enzyme if a natural product is used as starting material. In the cases where the natural product has too low an enzymatic activity or when IP₆, IP₅ or IP₄ or a mixture of these is used as starting material, a phytase enzyme, for example, from bran is added.

A suitable way to treat the natural or crude starting material is to pretreat it, for instance by breakage or removal of outer membrane and removal of unwanted constituents. Thus, when using pollen the allergens should be removed. Thereafter, the material is soaked in water to make the inositol phosphate available for breaking down and to activate the enzyme. In the cases where an extra quantity of enzymes is necessary, this quantity is added at this stage. The enzyme is then allowed to act for so long a time as is necessary for the intended degree of hydrolysis to be achieved.

The hydrolysis takes place at a suitable temperature, usually 20-70^{o}C, preferably 30-40^{o}C and at optimal pH-level for the phytase present. In order to stop the hydrolysis at the intended level the enzyme may be destroyed or inactivated, for instance by a rapid heating of the hydrolysed starting material. This also ensures that an uncontrolled and undesired continued hydrolysis of IP₃ in the stomach will not continue when the composition is administered. In order to transfer the material to a form which is stable at storage it can suitably be freeze dried.

Yeast can be used advantageously as a source of phytase. Preferably baker's yeast is used. When using yeast essentially only one isomer of IP₃ is obtained, namely D-myo-inositol-1.2.6-triphosphate.

The above mentioned procedure, in applicable parts with possible modifications, can be used also when one or more of the compounds IP₆, IP₅ or IP₄ per se are used as starting material.

The pharmaceutical composition according to the invention comprises as a pharmaceutically active ingredient at least one isomer of inositol triphosphate (IP₃).

It is suitable that the composition according to the invention exists in unit dosage form. Tablets, granulates or capsules are suitable administration forms for such unit dosage. Furthermore, tablets and granulates can easily be surface treated such as to provide an enteric coating to prevent an uncontrolled hydrolysis in the stomach and to bring about a desired absorption in the intestine. Other suitable administration forms are slow release and transdermal administration. A usual pharmaceutically acceptable additive, excipient and/or carrier can be included in the composition. The tablets or granulates can also contain a disintegrant which causes the tablets or the granulates, respectively, to disintegrate easily in the intestine. In certain cases, especially in acute situations, it is preferable to use the unit dosage in the form of a solution for intravenous administration.

The pharmaceutical composition can also consist as such of IP₃ solely without any additive, excipient or carrier.

If desired, the composition can be free of other inositol phosphates IP₁, IP₂, IP₄, IP₅ and IP₆. Accordingly, the mixture of IP₃ isomers can have a purity of 90-100%, such as 93-100% or preferably 95-100%.

Alternatively, the pharmaceutical composition can consist of or comprise one or more specific IP₃ isomers disclosed hereinafter, each present in substantially pure form. Thus, the different isomers can be isolated from each other in substantially pure form, which means that they have a purity of 80-100%, such as 82-100% or 85-100%, preferably 90-100%. Since the isomers can be produced in pure form they can be mixed in any proportion, of course.

The production of IP₃ and the isolation of the different isomers thereof are disclosed in the U.S. patent application 788,829 filed on October 18, 1985 and the equivalent U.K. patent application 2,169,602 for instance.

It is in most cases suitable that the IP₃-isomer or isomers in the composition according to the invention are present in salt form in order not to affect the mineral balance negatively. The salt should preferably consist of a sodium, calcium, zinc or magnesium salt or a mixture of two or more of these salts. Calcium and zinc salts or mixtures of these are especially preferred. The isomer of IP₃ can also partly be present as a salt of one or more physiologically acceptable compounds in the lanthanide series; i.e. La, Ce, Pr, Nd, Pm, Sm, Eu, Gd, Tb, Dy, Ho, Er, Tm, Yb and Lu.

For the above mentioned reasons it is also an advantage if the composition contains a surplus or an extra addition of at least one pharmaceutically acceptable salt of calcium, zinc or magnesium with a mineral acid or organic acid. This is especially valuable for older persons who are often deficient in these minerals.

The composition according to the present invention can preferably also contain at least one substance containing selenium, an unsaturated fatty acid, such as gamma linoleic acid, vitamin E, vitamin C or a pharmaceutically acceptable organic acid or salt thereof, such as citrate, oxalate, malonate and tartrate. These substances also help to counteract the negative effect of lead, mercury, nickel and/or chromium in the body and/or to give in addition thereto, in certian cases, a desirable effect together with the IP₃ isomer in the composition. The content of selenium in the composition is preferably such that the daily intake is about 0.7-8 µg/kg body weight preferably 0.7-3.3 µg/kg. For vitamin E the corresponding values are about 0.1-2 mg and 0.1-1 mg, respectively.

The composition is suitably free from penicillin.

The preferred dosage for humans falls within the range of 0.1 to 10 mg IP₃/day/kg body weight.

In animal experiments, no toxic effects were seen after administration of very high doses of IP₃, 160 mg/kg body weight by intravenous injection to mice or 1600 mg/kg body weight by intraperitoneal injection to mice.

The composition used in the present invention contains at least one, sometimes two or more of the following substances, which correspond to the essential IP₃-isomer or isomers mentioned above:
D-myo-inositol-1.2.6-triphosphate of the formula 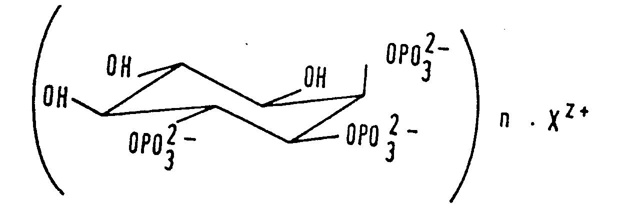 where X is hydrogen, at least one univalent, divalent or multivalent cation, or a mixture thereof, n is the number of ions, and z is the charge of the respective ion;
D-myo-inositol-1.2.5-triphosphate of the formula 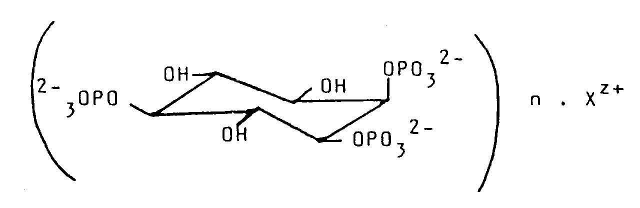 where X, n and z have the above mentioned meaning;
myo-inositol-1.2.3.-triphosphate of the formula 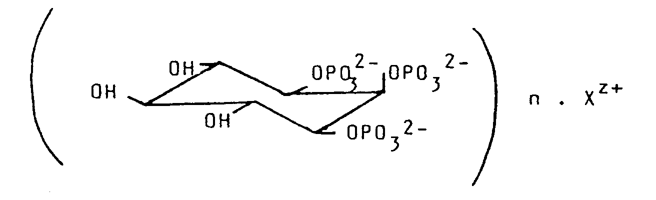 where X, n and z have the above mentioned meaning;
L-myo-inositol-1.3.4-triphosphate of the formula 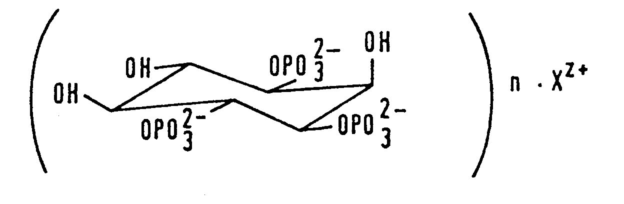 where X, n and z have the above mentioned meaning; and
D-myo-inositol-1.4.5-triphosphate of the formula 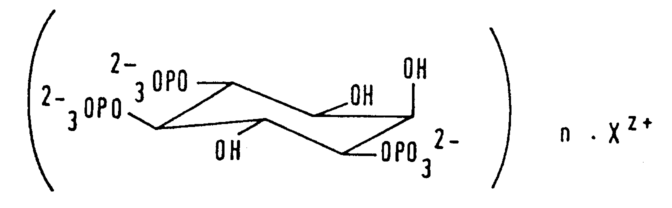 where X, n and z have the above mentioned meaning.

In each of the above formulas n ranges between 6 to 1 inclusive and z ranges from 1 to 6 inclusive. Preferably, n is between 3 to 6 inclusive and z is 3, 2 or 1. Of above isomers D-myo-inositol-1.2.6-triphosphate is preferred.

IP₃ may be the sole pharmaceutically active ingredient in the composition. However, also other pharmaceutically active ingredients can be present therein. The amount of IP₃ should then constitute 5 to 95 or 15 to 80, such as 25 to 60 per cent by weight of said active ingredients.

Moreover, the composition can be a multi-vitamin unit containing 2 to 60, such as 2 to 40 or preferably 2 to 25 per cent by weight of IP₃ based on the total weight of pharmaceutically active ingredients.

The composition usually contains 0.01-1.5 g, such as 0.05-1.3 or preferably 0.1-1 g of IP₃.

The invention also comprises a method of preventing or alleviating inflammatory conditions.

The method comprises administering to a human or an animal an amount of inositol triphosphate sufficient to obtain said prevention or alleviation.

The invention is further explained below in connection with embodiment examples of which examples 1-7 show the production of IP₃ and separation thereof into different isomers. In example 8 the manufacture of a solution of a potassium salt of D-myo-inositol-1.2.6-triphosphate for injection is shown. Example 9 finally describes manufacture of tablets of a calcium salt of D-myo-inositol-1.2.6-triphosphate.

### Example 1

### Hydrolysis of sodium phytate with wheat phytase and fractionation of a mixture of inositolphosphates.

A 1.6 gram quantity of sodium phytate (from corn, Sigma Chemical Co.) was dissolved in 650 ml sodium acetate buffer, pH 5.2. 2.7 gram wheat phytase (EC 3.1.3.26, 0.015 U/mg, from Sigma Chemical Co.) was added and the mixture was incubated at 38^{o}C.

The dephosphorylation was followed by determining the inorganic phosphorus released. After 3 hours when 50% inorganic phosphorus was liberated the hydrolysis was stopped by adding 30 ml ammonia to pH 12. A liquid mixture containing inositolphosphates was obtained.

350 ml of the mixture was passed through an ion-exchange column (Dowex 1, chloride form, 25 mm x 250 mm) and eluted with a linear gradient of hydrochloric acid (0-0.7 N HCl). Aliquots of eluted fractions were completely hydrolyzed in order to determine the contents of phosphorus and inositol. The peaks correspond to different inositolphosphates, i.e. a peak with the ratio of phosphorus to inositol of three to one consists of inositoltriphosphate etc. Two fractions with the ratio of phosphorus to inositol of three to one were obtained.

### Example 2

### Fractionation of inositoltriphosphates.

100 ml of the first fraction obtained in Example 1 with a phosphorus/inositol ratio of three to one was neutralized and precipitated as a bariumsalt after addition of 10% excess of 0.1 M bariumacetate solution. 600 mg of the precipitated salt was dissolved in 50 ml diluted hydrochloric acid. The solution was separated on an ion-exchange column (Dowex 1, chloride form, 25 mm x 2500 mm) with diluted hydrochloric acid as eluent. Aliquots of eluted fractions were analyzed for phosphorus. Three peaks consisting of isomers of inositoltriphosphates could be seen.

### Example 3

### Structural determination of isomers of inositoltriphosphates with NMR.

The three peaks obtained in Example 2 were analyzed by H-NMR. Data show that the peaks consist of myo-inositol-1.2.6-triphosphate, myo-inositol-1.2.3-triphosphate and myo-inositol-1.3.4-triphosphate respectively.

The second fraction obtained in Example 1 with a phosphorus/inositol ratio of three to one was analyzed by H-NMR. Data show that the fraction consists of myo-inositol-1.2.5-triphosphate.

### Example 4

### Determination of optical isomers of inositoltriphosphate.

20 mg of the compounds determined with NMR according to Example 3 to be myo-inositol-1.2.6-triphosphate and myo-inositol-1.3.4-triphosphate were further chromatographed on a chiral column based on acetylated cellulose (20 mm x 300 mm from Merck) with a mixture of ethanol and water as eluent. The fractions were analyzed with a polarimeter. Each compound consists of one optical isomer, D-myo-inositol-1.2.6-triphosphate and L-myo-inositol-1.3.4-triphosphate respectively.

### Example 5

### Hydrolysis of sodium phytate with baker's yeast and fractionation of a mixture of inositolphosphates.

A 0.7 gram quantity of sodium phytate (from corn, Sigma Chemical Co.) was dissolved in 600 ml sodium acetate buffer pH 4.6. 50 gram of baker's yeast from Jästbolaget, Sweden (dry substance: 28%, nitrogen content: 2%, phosphorus content: 0.4%) was added with stirring and incubation was continued at 45^{o}C. The dephosphorylation was followed by determining the inorganic phosphorus released. After 7 hours when 50% inorganic phosphorus was liberated the hydrolysis was stopped by adding 30 ml of ammonia to pH 12. The suspension was centrifuged and the supernatent was collected.

400 ml of the supernatent was passed through an ion-exchange column (Dowex 1, chloride form, 25 mm x 250 mm) and eluted with a linear gradient of hydrochloric acid (0-0.7 N HCl).

Aliquots of eluted fractions were completely hydrolyzed in order to determine the contents of phosphorus and inositol. The peaks correspond to different inositolphosphates, i.e. a peak with the ratio of phosphorus to inositol of three to one consists of inositoltriphosphates etc.

### Example 6

### Structural determination of isomers of inositoltriphosphate.

The fraction obtained in Example 5 with a phosphorus/inositol ratio of three to one was neutralized and evaporated before analysis with H-NMR. Data show that the peak consists of myo-inositol-1.2.6-triphosphate.

### Example 7

### Determination of optical isomers of myo-inositol-triphosphate.

The same method was used as described in Example 4 with the difference that 10 mg of the compound determined with NMR according to Example 10 was analyzed. The compound consists of one optical isomer, D-myo-inositol-1.2.6-triphosphate.

### Example 8

### Solution of potassiumsalt of D-myo-inositol-1.2.6-triphosphate for injection.

0.5 g of the potassiumsalt of IP₃ and 0.77 g NaCl were dissolved in 98.73 ml of water for injection to form a solution suitable for injection into a person or an animal.

### Example 9

### Tablets of calciumsalt of D-myo-inositol-1.2.6-triphosphate.

Tablets of the calciumsalt of D-myo-inositol-1.2.6-triphosphate were produced in the following way. 50 g calciumsalt of D-myo-inositol-1.2.6-triphosphate, 132 g lactose and 6 g acacia were mixed. Purified water was then added to the mixture, whereupon the mixing was continued until a suitable consistency was obtained. The mixture was sieved and dried. Then the mixture was blended with 10 g talcum and 2 g magnesium stearate. The mixture was compressed into tablets each weighing 200 mg.

For purposes of further understanding the invention, formulas are given below of the IP₃ isomers of the invention. Formulas are also given for IP₆, IP₅, IP₄ and IP₂.

The lower phosphate-esters of myoinositol are named depending on where the phosphoric acid groups are situated on the inositol ring, with the numbering giving as low position numbers as possible. L and D stand for clock-wise and counterclock-wise counting respectively, and are used depending on which result gives the lowest position number. The carbon atom which has an axial phosphoric acid group always has the position number 2. The structural formulas below are simplified to the acid form.

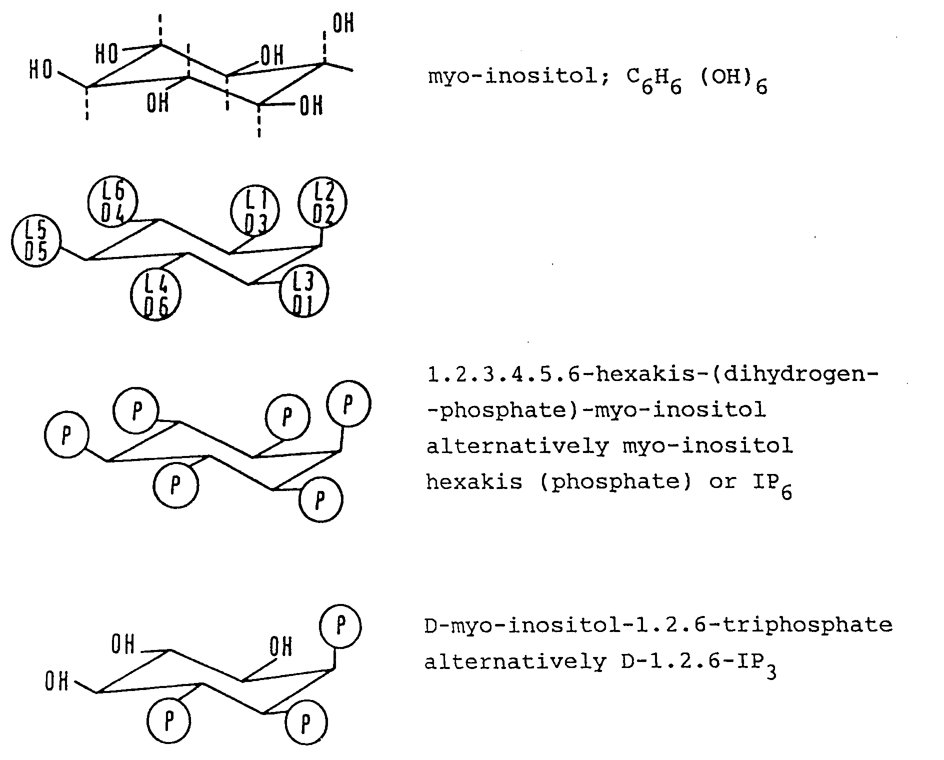

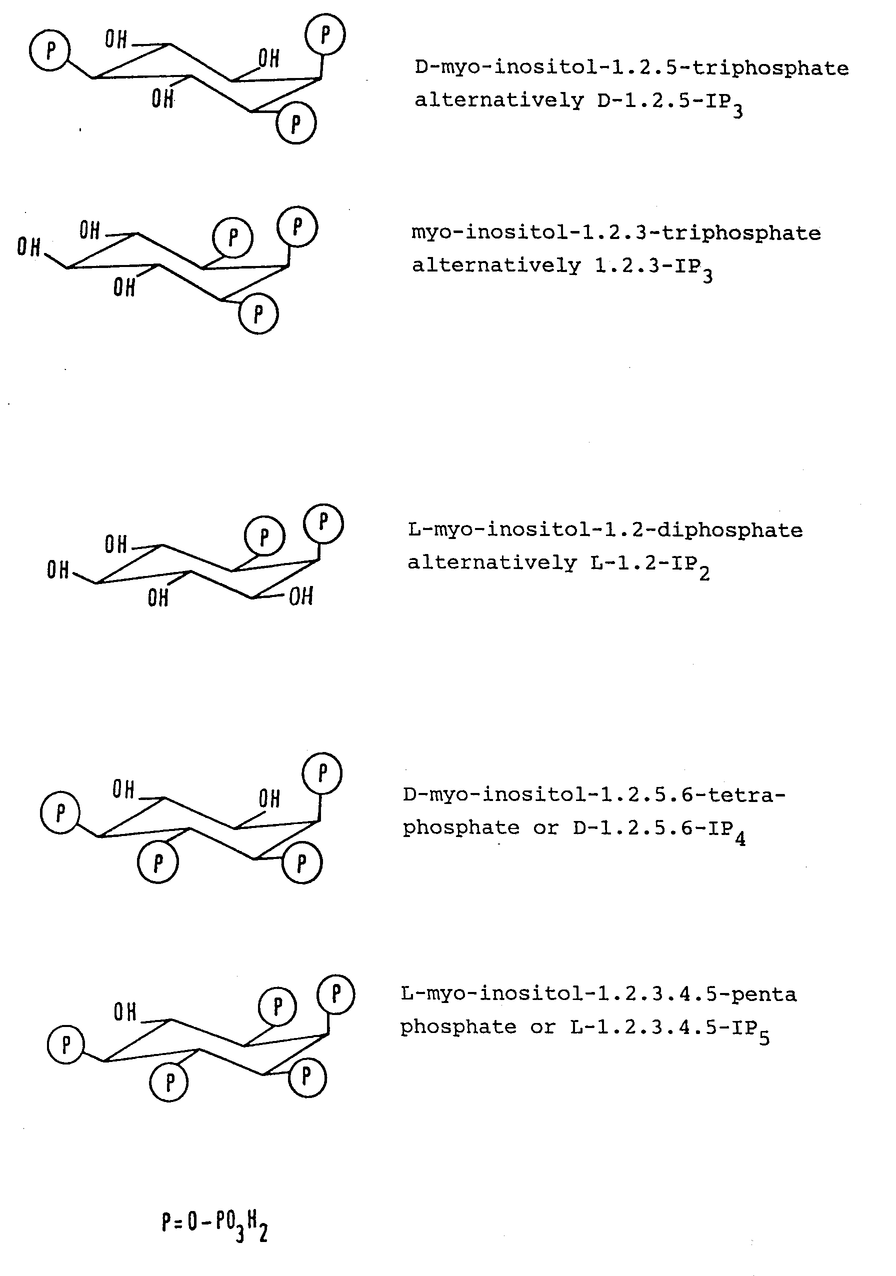

## Claims

1. The use of inositol triphosphate for the preparing of a medicament for preventing or alleviating inflammatory conditions.

2. The use according to claim 1, wherein said inositoltriphosphate is in salt form.

3. The use according to claim 2, wherein said inositoltriphosphate salt is a salt of sodium, calcium, zinc or magnesium or a mixture of two or more thereof.

4. The use according to any one of claims 1-3, wherein the medicament is in tablet or granulated form.

5. The use according to any one of claims 1-3, wherein the medicament is in the form of a solution.

6. The use according to any one of claims 1-5, wherein the medicament is further comprising a pharmaceutically acceptable salt of mineral acid or organic acid with at least one of calcium, zinc or magnesium.

7. The use according to any one of claims 1-6, wherein the medicament is further comprising at least one additive selected from the group consisting of a selenium compound, an unsaturated fatty acid such as gamma linolenic acid, vitamin E, vitamin C and a pharmaceutically acceptable organic acid or salt thereof.

8. The use according to claim 7, wherein said salt is a citrate, oxalate, malonate or tartrate.

9. The use according to any one of claims 1-8, wherein the medicament is free from penicillin.

10. The use according to any one of claims 1-9, wherein the medicament is containing at least one other pharmaceutically active ingredient in addition to IP₃.

11. The use according to claim 10, wherein the amount of IP₃ is in the range of 5-95 per cent by weight of the active ingredients.

12. The use according to claim 10, wherein the medicament is a multi-vitamin unit containing 2-60 per cent by weight of IP₃ based on the total weight of pharmaceutically active ingredients.

13. The use according to any one of claims 1-12, wherein the medicament is containing 0.01-1.5 g of IP₃.

14. The use according to any one of claims 1-13, wherein IP₃ has the formula 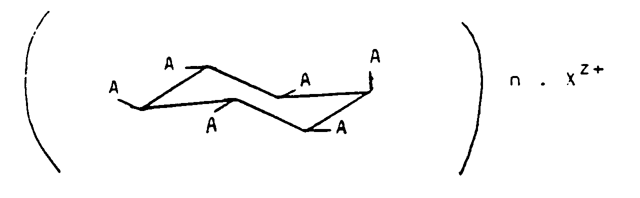 where three A is OH and three A is OPO₃²⁻,
X is hydrogen and/or at least one univalent, divalent or multivalent cation; n is the number of ions; and z is the charge of the respective ions.

15. The use according to claim 14, wherein n ranges between 6 to 1 inclusive, z ranges between 1 to 6 inclusive and n is preferably between 3 to 6 inclusive and z is 3, 2 or 1.

16. The use according to claim 14, wherein said IP₃ comprises D-myo-inositol-1.2.6-triphosphate with the formula 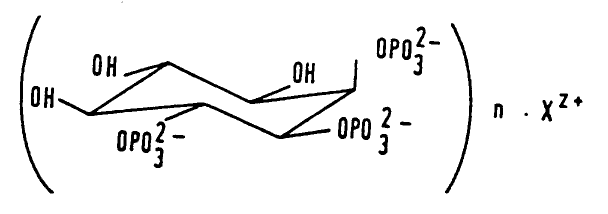 where X is hydrogen or at least one univalent, divalent or multivalent cation; n is the number of ions; and z is the charge of the respective ion.

17. The use according to claim 14, wherein said IP₃ comprises D-myo-inositol-1.2.5-triphosphate with the formula 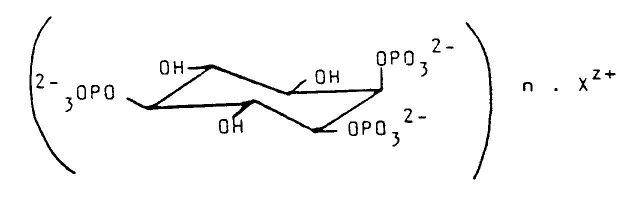 where X is hydrogen or at least one univalent, divalent or multivalent cation; n is the number of ions; and z is the charge of the respective ion.

18. The use according to claim 14, wherein said IP₃ comprises myo-inositol-1.2.3-triphosphate with the formula 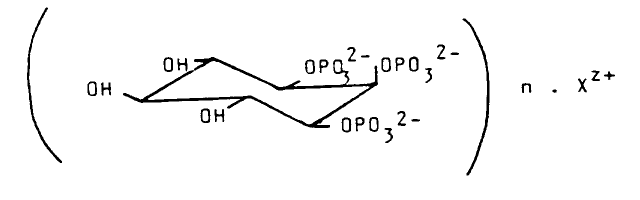 where X is hydrogen or at least one univalent, divalent or multivalent cation; n is the number of ions; and z is the charge of the respective ion.

19. The use according to claim 14, wherein said IP₃ comprises L-myo-inositol-1.3.4-triphosphate with the formula 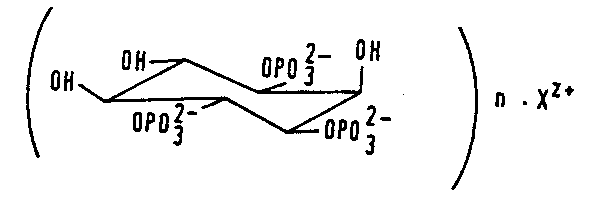 where X is hydrogen and/or at least one univalent, divalent or multivalent cation; n is the number or ions; and z is the charge of the respective ion.

20. The use according to claim 14, wherein said IP₃ comprises D-myo-inositol-1.4.5-triphosphate with the formula 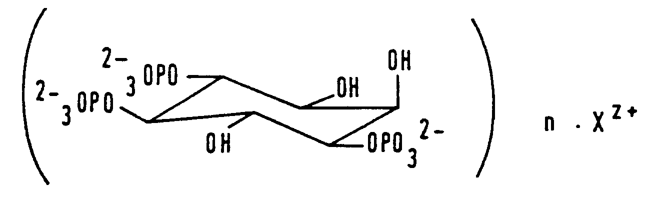 where X is hydrogen and/or at least one univalent, divalent or multivalent cation; n is the number of ions; and z is the charge of the respective ion.

21. The use according to any one of claims 1-20, wherein the medicament is in a pharmaceutical unit dosage form and further comprising a pharmaceutically acceptable carrier, excipient or additive.

## Patentansprüche

1. Verwendung von Inosittriphosphat für die Herstellung eines Medikaments zur Verhütung oder Linderung von entzündlichen Zuständen.

2. Verwendung gemäss Anspruch 1, worin das Inosittriphosphat in Salzform vorliegt.

3. Verwendung gemäss Anspruch 2, worin das Inosittriphosphatsalz ein Natriumsalz, Calciumsalz, Zinksalz oder Magnesiumsalz oder eine Mischung von zwei oder mehreren davon ist.

4. Verwendung gemäss einem der Ansprüche 1 bis 3, worin das Medikament in Tablettenform oder granulierter Form vorliegt.

5. Verwendung gemäss einem der Ansprüche 1 bis 3, worin das Medikament in Form einer Lösung vorliegt.

6. Verwendung gemäss einem der Ansprüche 1 bis 5, worin das Medikament weiterhin ein pharmazeutisch annehmbares Salz einer Mineralsäure oder organischen Säure mit wenigstens einem Vertreter aus Calcium, Zink oder Magnesium umfasst.

7. Verwendung gemäss einem der Ansprüche 1 bis 6, worin das Medikament weiterhin wenigstens einen Zusatzstoff umfasst, ausgewählt aus einer Selenverbindung, einer ungesättigten Fettsäure wie γ-Linolsäure, Vitamin E, Vitamin C und einer pharmazeutisch annehmbaren organischen Säure oder deren Salz.

8. Verwendung gemäss Anspruch 7, worin das Salz ein Citrat, Oxalat, Malonat oder Tartrat ist.

9. Verwendung gemäss einem der Ansprüche 1 bis 8, worin das Medikament penicillinfrei ist.

10. Verwendung gemäss einem der Ansprüche 1 bis 9, worin das Medikament wenigstens einen anderen pharmazeutischen Wirkstoff zusätzlich zu IP₃ enthält.

11. Verwendung gemäss Anspruch 10, worin die IP₃-Menge im Bereich von 5 bis 95 Gew.% der Wirkstoffe liegt.

12. Verwendung gemäss Anspruch 10, worin das Medikament eine Multivitamineinheit ist, enthaltend 2 bis 60 Gew.% IP₃, bezogen auf das Gesamtgewicht der pharmazeutischen Wirkstoffe.

13. Verwendung gemäss einem der Ansprüche 1 bis 12, worin das Medikament 0,01 bis 1,5 g IP₃ enthält.

14. Verwendung gemäss einem der Ansprüche 1 bis 13, worin IP₃ die Formel 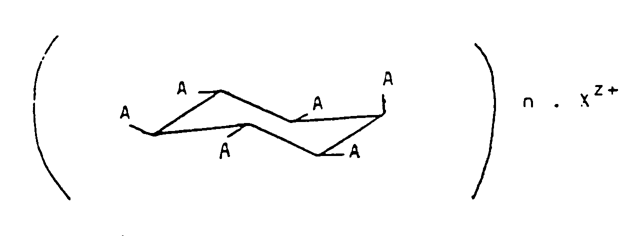 hat, wobei drei A OH sind und drei A OPO₃²⁻ sind, X Wasserstoff und/oder wenigstens ein einwertiges, zweiwertiges oder mehrwertiges Kation ist; n die Anzahl der Ionen ist; und z die Ladung der jeweiligen Ionen ist.

15. Verwendung gemäss Anspruch 14, worin n zwischen 6 und 1 einschliesslich liegt, z zwischen 1 und 6 einschliesslich liegt und n bevorzugt zwischen 3 und 6 einschliesslich ist und z 3, 2 oder 1 ist.

16. Verwendung gemäss Anspruch 14, worin das IP₃ D-myo-Inosit-1,2,6-triphosphat mit der Formel 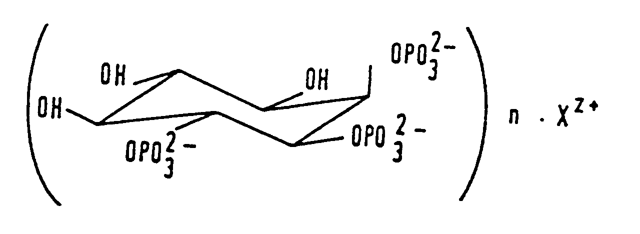 umfasst, wobei X Wasserstoff oder wenigstens ein einwertiges, zweiwertiges oder mehrwertiges Kation ist; n die Anzahl der Ionen ist; und z die Ladung des jeweiligen Ions ist.

17. Verwendung gemäss Anspruch 14, worin das IP₃ D-myo-Inosit-1,2,5-triphosphat mit der Formel 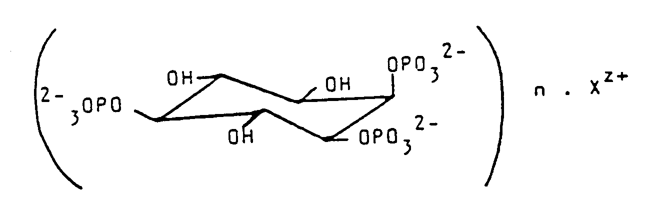 umfasst, wobei X Wasserstoff oder wenigstens ein einwertiges, zweiwertiges oder mehrwertiges Kation ist; n die Anzahl der Ionen ist; und z die Ladung des jeweiligen Ions ist.

18. Verwendung gemäss Anspruch 14, worin das IP₃ myo-Inosit-1,2,3-triphosphat mit der Formel 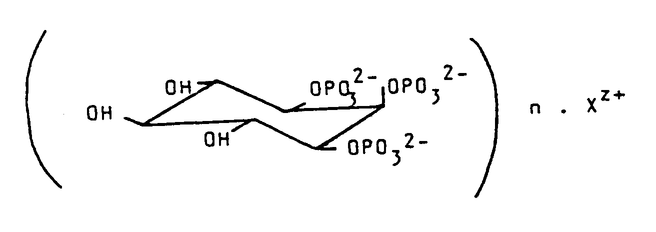 umfasst, wobei X Wasserstoff oder wenigstens ein einwertiges, zweiwertiges oder mehrwertiges Kation ist; n die Anzahl der Ionen ist; und z die Ladung des jeweiligen Ions ist.

19. Verwendung gemäss Anspruch 14, worin das IP₃ L-myo-Inosit-1,3,4-triphosphat mit der Formel 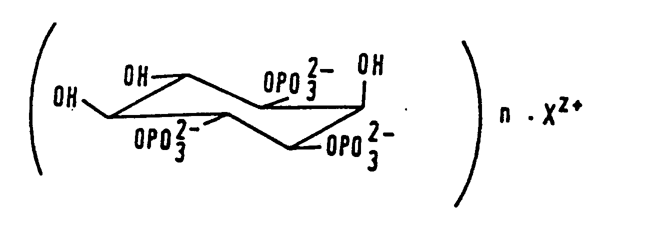 umfasst, wobei X Wasserstoff und/oder wenigstens ein einwertiges, zweiwertiges oder mehrwertiges Kation ist; n die Anzahl der Ionen ist; und z die Ladung des jeweiligen Ions ist.

20. Verwendung gemäss Anspruch 14, worin das IP₃ D-myo-Inosit-1,4,5-triphosphat mit der Formel 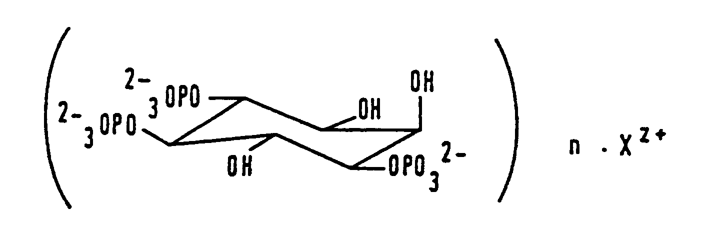 umfasst, wobei X Wasserstoff und/oder wenigstens ein einwertiges, zweiwertiges oder mehrwertiges Kation ist; n die Anzahl der Ionen ist; und z die Ladung des jeweiligen Ions ist.

21. Verwendung gemäss einem der Ansprüche 1 bis 20, worin das Medikament in einer pharmazeutischen Einheitsdosisform vorliegt und weiterhin einen pharmazeutisch annehmbaren Trägerstoff, Träger oder Zusatzstoff umfasst.

## Revendications

1. Utilisation de triphosphate d'inositol pour la préparation d'un médicament pour éviter ou soulager des états inflammatoires.

2. Utilisation selon la revendication 1, dans laquelle ledit triphosphate d'inositol est sous la forme d'un sel.

3. Utilisation selon la revendication 2, dans laquelle ledit sel de triphosphate d'inositol est un sel de sodium, de calcium, de zinc ou de magnésium ou un mélange de deux ou plus de ceux-ci.

4. Utilisation selon l'une quelconque des revendications 1-3, dans laquelle le médicament est sous la forme de comprimés ou de granulés.

5. Utilisation selon l'une quelconque des revendications 1-3, dans laquelle le médicament est sous la forme d'une solution.

6. Utilisation selon l'une quelconque des revendications 1-5, dans laquelle le médicament comprend en outre un sel pharmaceutiquement acceptable d'un acide inorganique ou d'un acide organique, avec au moins l'un du calcium, du zinc ou du magnésium.

7. Utilisation selon l'une quelconque des revendications 1-6, dans laquelle le médicament comprend en outre au moins un additif choisi dans le groupe constitué d'un composé du sélénium, d'un acide gras insaturé tel qu'un acide gamma linolénique, la vitamine E, la vitamine C et un acide organique pharmaceutiquement acceptable ou un sel de celui-ci.

8. Utilisation selon la revendication 7, dans laquelle ledit sel est un citrate, un oxalate, un malonate ou un tartrate.

9. Utilisation selon l'une quelconque des revendications 1-8, dans laquelle le médicament est exempt de pénicilline.

10. Utilisation selon l'une quelconque des revendications 1-9, dans laquelle le médicament contient au moins un autre ingrédient pharmaceutiquement actif en plus de l'IP₃.

11. Utilisation selon la revendication 10, dans laquelle la quantité de l'IP₃ est dans la gamme 5-95 pour cent en poids des ingrédients actifs.

12. Utilisation selon la revendication 10, dans laquelle le médicament est une unité multivitamines contenant 2-60 pour cent en poids d'IP₃ par rapport au poids total des ingrédients pharmaceutiquement actifs.

13. Utilisation selon l'une quelconque des revendications 1-12, dans laquelle le médicament contient 0,01-1,5 g d'IP₃.

14. Utilisation selon l'une quelconque des revendications 1-13, dans laquelle l'IP₃ a la formule : 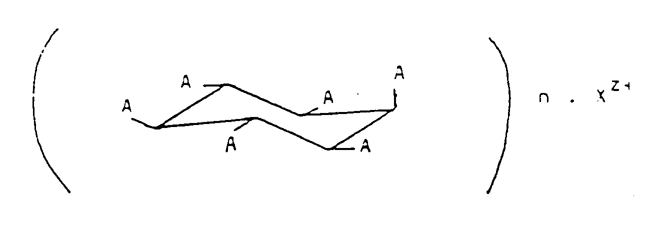 dans laquelle trois A sont OH et trois A sont OPO₃²⁻, X est un atome d'hydrogène et/ou au moins un cation univalent, divalent ou multivalent; n est le nombre d'ions; et z est la charge des ions respectifs.

15. Utilisation selon la revendication 14, dans laquelle n est compris entre 6 et 1 inclus, z est compris entre 1 et 6 inclus et n est de préférence compris entre 3 et 6 inclus et z est 3, 2 ou 1.

16. Utilisation selon la revendication 14, dans laquelle ledit IP₃ comprend du D-myo-inositol-1,2,6-triphosphate avec la formule : 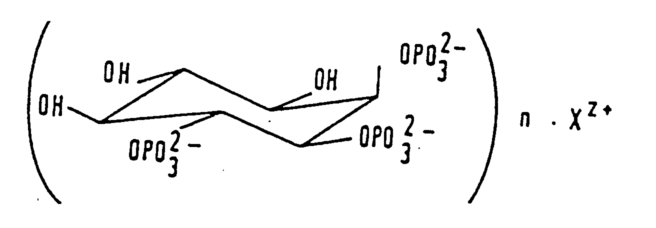 dans laquelle X est un atome d'hydrogène ou au moins un cation univalent, divalent ou multivalent; n est le nombre d'ions; et z est la charge de l'ion respectif.

17. Utilisation selon la revendication 14, dans laquelle ledit IP₃ comprend du D-myo-inositol-1,2,5-triphosphate avec la formule : 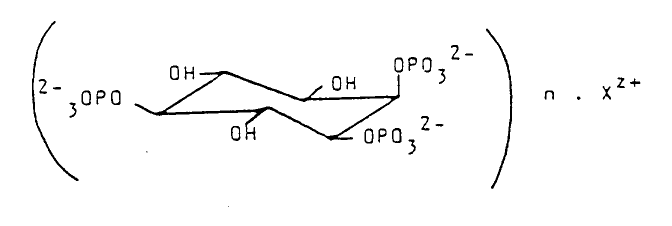 dans laquelle X est un atome d'hydrogène ou au moins un cation univalent, divalent ou multivalent; n est le nombre d'ions; et z est la charge de l'ion respectif.

18. Utilisation selon la revendication 14, dans laquelle ledit IP₃ comprend du D-myo-inositol-1,2,3-triphosphate avec la formule : 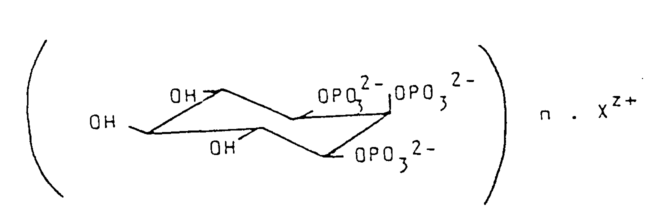 dans laquelle X est un atome d'hydrogène ou au moins un cation univalent, divalent ou multivalent; n est le nombre d'ions; et z est la charge de l'ion respectif.

19. Utilisation selon la revendication 14, dans laquelle ledit IP₃ comprend du L-myo-inositol-1,3,4-triphosphate avec la formule : 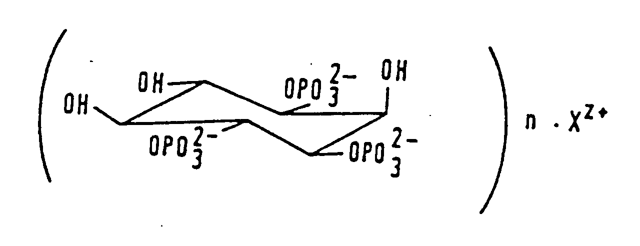 dans laquelle X est un atome d'hydrogène et/ou au moins un cation univalent, divalent ou multivalent; n est le nombre d'ions; et z est la charge de l'ion respectif.

20. Utilisation selon la revendication 14, dans laquelle ledit IP₃ comprend du D-myo-inositol-1,4,5-triphosphate avec la formule : 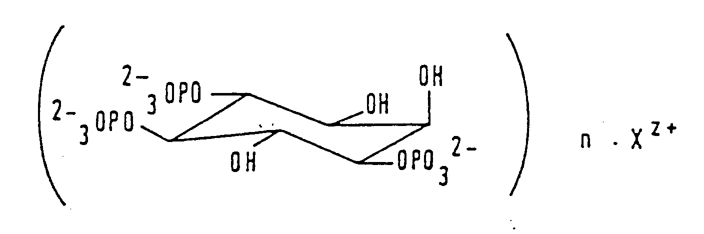 dans laquelle X est un atome d'hydrogène et/ou au moins un cation univalent, divalent ou multivalent; n est le nombre d'ions; et z est la charge de l'ion respectif.

21. Utilisation selon l'une quelconque des revendications 1-20, dans laquelle le médicament est sous la forme d'un dosage pharmaceutique unitaire et comprend en outre un support, un excipient ou un additif pharmaceutiquement acceptables.
